# EUROPEAN PATENT SPECIFICATION

(11) **EP 0 873 308 B1**
(45) Date of publication and mention of the grant of the patent: **02.01.2002**
(21) Application number: 96935553.6
(22) Date of filing: 10.10.1996
(51) Int. Cl.: C07C 271/12

(54) **O-CARBAMOYL-PHENYLALANINOL COMPOUNDS AND THEIR PHARMACEUTICALLY USEFUL SALTS**
O-CARBAMOYL-PHENYLALANINOL-ZUSAMMENSETZUNGEN UND IHRE PHARMAZEUTISCH ANWENDBAREN SALZEN
COMPOSES DE O-CARBAMOYL-PHENYLALANINOL ET SELS DE CES COMPOSES UTILES SUR LE PLAN PHARMACEUTIQUE

(43) Date of publication of application: 28.10.1998
(73) Proprietor: SK Corporation, Yongdungpo-ku, Seoul 150-010 (KR)
(72) Inventor: CHOI, Yong, Moon, Towaco, NY 07082 (US); HAN, Dong, Il, Yusung-ku Taejon 305-390 (KR); KIM, Yong, Kil, Yusung-ku Taejon 305-390 (KR); SHIN, Hun, Woo, Yusing-ku Taejon 305-390 (KR); PARK, Jeong, Han, Seoul 136-045 (KR)
(74) Representative: Perani, Aurelio
(86) International application number: KR9600174
(87) International publication number: WO9815526

(56) References cited:
- WO-A-96/24577
- US-A- 2 937 119

## Description

The present invention relates, in general, to novel phenylalkylamino carbamate compounds and pharmaceutically useful salts thereof for treating diseases of the central nervous system. More particularly, the present invention relates to racemic mixtures or enantiomerically enriched O-carbamoyl-phenylalaninol compounds and pharmaceutically useful salts thereof.

### Description of the Prior Art

Phenylethylamine derivatives, one important class of therapeutical medicines useful for managing central nervous system (CNS) diseases, have been used mainly to treat obesity, narcolepsy, minimal brain dysfunction and mild depression.

Organic carbamates have been effectively used for controlling various CNS disorders. For example, the J. Am. Chem. Soc., 73, 5779 (1951) discloses 2-methyl-2-propyl-1,3-propandiol dicarbamate and its pharmaceutical activity was verified in J. Pharmacol. Exp. Ther., 104,229 (1952).

In addition, there are many carbamate compounds that are suggested as therapeutics for CNS diseases in the prior art. For example, U. S. Pat. Nos. 2,884,444 and 2,937,119 disclose carbamates, such as 2-phenyl-1,3-propandiol dicarbamate and isopropylmeprobamate, respectively. These compounds are found to be very effectively used as therapeutics for treating CNS disorders, especially as antiepileptics and centrally acting muscle relaxants. Research in the development of carbamate therapeutics for CNS diseases has been and continues to be actively pursued.

The International Patent Application with publication no. WO 96/24577 describes O-carbamoyl-(D)-phenylalaninol compounds in which the phenyl group is unsubstituted. These compounds are described to be useful for the prophylaxis and treatment of CNS disorder.

The recent design of pharmacologically useful compounds has been based on amino acids or the derivatives thereof, which is mainly attributable to the fact that many of the compounds found in biological systems come from amino acids or the derivatives thereof. In addition, in most cases, the function of a pharmaceutically useful compound is effected after it binds to an enzyme or receptor, which may trigger the regulatory mechanisms of the enzyme or receptor.

### SUMMARY OF THE INVENTION

As a result of intensive and thorough research, the present inventors found that. O-carbamoyl-phenylalaninol compounds are pharmaceutically useful for CNS disorders, especially for depression and anxiety.

Accordingly, it is a principal object of the present invention to provide racemic or enantiomerically enriched O-carbamoyl-phenylalaninol carbamate compounds, represented by the following structural formula V: 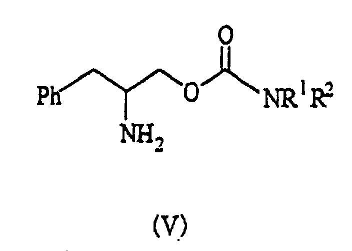 wherein Ph is a phenyl group as described as follows: 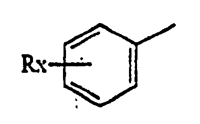 wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, and thioalkoxy containing 1 to 3 carbon atoms, and x is an integer from 1 to 3, with the proviso that R is the same or different when x is 2 or 3, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and R¹ and R² may be joined to form a 5 to 7-membered cyclic moiety which may contain one additional nitrogen atom substituted with a member selected from the group consisting of hydrogen, alkyl, and aryl groups, or one oxygen atom and the pharmaceutically acceptable salts thereof.

More specifically, it is a principal object of the present invention to provide O-carbamoyl-(D)-phenylalaninol compounds, represented by the following structural formula IX (alternatively, "D" can be referred to as R-configuration at chiral center in structural formula IX): 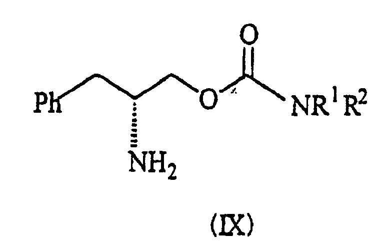 wherein Ph is a phenyl group as described as follows: 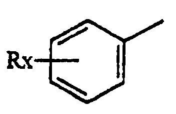 wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, and thioalkoxy containing 1 to 3 carbon atoms, and x is an integer from 1 to 3, with the proviso that R is the same or different when x is 2 or 3, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and R¹ and R² may be joined to form a 5 to 7-membered cyclic moiety which may contain one additional nitrogen atom substituted with a member selected from the group consisting of hydrogen, alkyl, and aryl groups, or one oxygen atom and the pharmaceutically acceptable salts thereof.

### DETAILED DESCRIPTION OF THE INVENTION

In accordance with the present invention, the racemic or enantiomerically enriched O-carbamoyl-phenylalaninols represented by the structural formula V and pharmaceutically acceptable salts thereof can be prepared by the following steps starting from readily available starting materials represented by the following structural formula II: 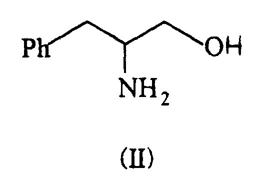 wherein Ph is the same as defined above.

It should be noted that the stereochemistry of the final products (V and IX) depends solely on that of the starting material II, for example, a starting material II with D-enantiomer yields only a product with D-enantiomer IX.

The first method for preparing the novel compounds of the general structural formula V will be described in detail below.

Phenylalaninols of structural formula II are reacted with benzyl chloroformate in a basic aqueous solution to synthesize N-benzyloxycarbonyl-phenylalaninol, represented by the following structural formula III: 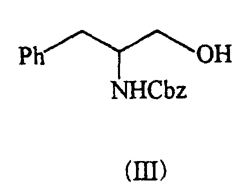 wherein Cbz is benzyloxycarbonyl group. Then, N-benzyloxycarbonyl-phenylalaninol of structural formula III is subjected to carbamoylation with phosgene (or 1, 1'-carbonyldiimidazole) in the presence of an amine base, represented by the following general formula VI:

R¹R²NH (VI)

wherein R¹ and R² are the same as defined above, to produce O-carbamoyl-N-benzyloxycarbonyl-phenylalaninol, represented by the following structural formula IV: 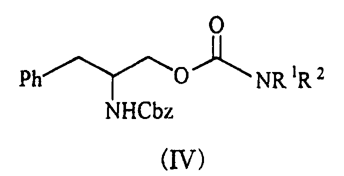 wherein R¹ and R² are the same as defined above, deprotecting the benzyloxycarbonyl group from O-carbamoyl-N-benzyloxycarbonylphenylalaninol of the structural formula IV through hydrogenolysis in the presence of a catalyst, to yield O-carbamoyl-phenylalaninol compound, represented by the structural formula V as shown above, wherein R¹ and R² are the same as defined above; and treating O-carbamoyl-(D)-phenylalaninol compound of structural formula V with an anhydrous acid, in an ethereal solution without further purification, to give a pharmaceutically acceptable salt thereof, represented by the following structural formula I: 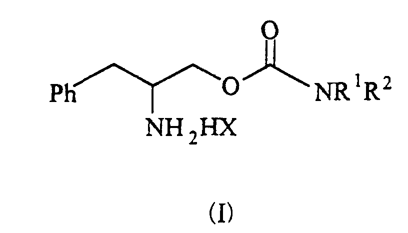 wherein R¹ and R² are the same as defined above and HX is an acid capable of forming a pharmaceutically useful salt with the intramolecular basic nitrogen atom.

This procedure for preparing the compound of structural formula I is summarized in Reaction Scheme I set forth below.

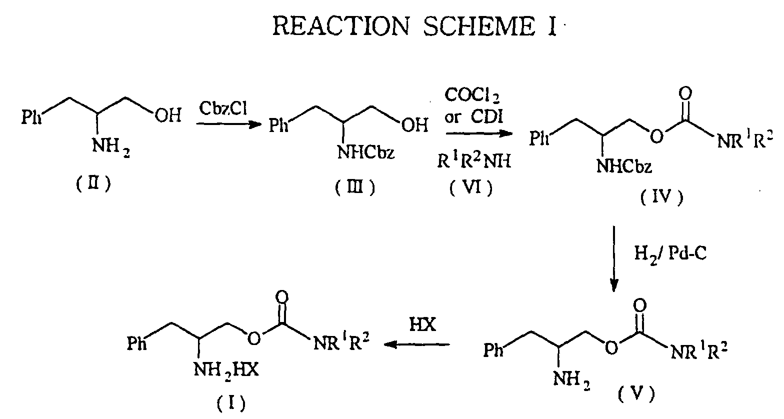

As shown in Reaction Scheme I, phenylalaninol II is first reacted with benzyl chloroformate in a basic aqueous solution, to give N-benzyloxycarbonyl- phenylalaninol III, which is subjected to carbamoylation with phosgene in the presence of an amine base. Ammonolysis of the carbamoylated intermediate is carried out and an amine represented by the general formula VI is used to produce O-carbamoyl-N-benzyloxycarbonyl-phenylalaninol IV in a high yield within a short period of time. Removal of the benzyloxycarbonyl group, a nitrogen protecting group, from the O-carbamoyl-N-benzyloxycarbonyl-phenyl alaninol IV through hydrogenolysis in the presence of a catalyst, affords O-carbamoyl-phenylalaninol V which is, then, treated with an anhydrous acid (HX) in an ether solution without further purification, to provide the salts I of O-carbamoyl-phenylalaninol. In Reaction Scheme I, HX represents an acid suitable for the formation of pharmaceutically acceptable salts with the intramolecular basic nitrogen atom.

Details of the reaction conditions described in Reaction Scheme I are as follows. In the first step, the concentration of the starting material II is between about 0.1 and 3.0 mole and benzyl chloroformate is used at about 1 to about 2 equivalents. The basic aqueous solution has a pH value between about 7 and about 14 and the conversion reaction is carried out at temperatures ranging from about -10 to about 70°C.

For the conversion of the compound III to the compound IV, about 1 to about 2 molar equivalent of phosgene, either neat or as a solution in toluene, is used at about 0.01 to about 2 molar concentration of the compound III. Halogenated alkanes such as methylene chloride, aromatic solvents, such as toluene, or mixtures thereof, can be used as a solvent. Use of a base such as acid scavenger is recommended. Typically, a tertiary amine, such as triethylamine, diisopropylethylamine, triisopropylamine, DBU (1,6-diazabicyclo [5.4.0]undec-7-ene), DBN (1,5-diazabicyclo[4.3.0]non-5-ene), antipyrine and dimethylphenylamine, can be used for this purpose. The reacting amine can be used neat, or as solution in water, or lower alkyl alcohol, such as methanol, ethanol, n-propanol and isopropanol and 1 to 2 molar equivalents is used. The conversion reaction is carried out at temperatures ranging from about -30 to about 60°C.

As for the hydrogenation from the compound IV to the compound V, an ethereal solvent such as THF, an alcoholic solvent, such as methanol, water, an aromatic solvent, such as toluene, benzene or xylene, an ester solvent, such as ethyl acetate or any compositional mixture thereof is used as a reaction medium. The hydrogenation from the compound IV to the compound V is carried out at a temperature of about -10 to about 150°C under about 1 to about 100 atm hydrogen pressure. This reaction is performed in the presence of a catalyst, such as palladium, platinum, platinum oxide, rhodium, and iridium.

Concrete examples of the anhydrous acid used for the preparation of the compound I from the compound V include hydrochloric acid, sulfuric acid, phosphoric acid, acetic acid, benzoic acid, citric acid, malonic acid, salicylic acid, malic acid, fumaric acid, oxalic acid, succinic acid, tartaric acid, lactic acid, gluconic acid, ascorbic acid, maleic acid, aspartic acid, benzene sulfonic acid, methane sulfonic acid, ethane sulfonic acid, hydroxymethane sulfonic acid and hydroxyethane sulfonic acid and the like. For additional acids, one can refer to "Pharmaceutical Salts", J. Pharm. Sci., 1977; 66(1): 1-19. This preparation is executed in a reaction media which can be exemplified by an ethereal solvent such as THF, an alcoholic solvent such as methanol, an ester solvent such as ethyl acetate, an aromatic solvent, and any compositional mixture thereof. An ethereal solvent is recommended as an addition solution, including ethyl ether, propyl ether, isopropyl ether, butyl ether, and isobutyl ether. The concentration of the compound V is on the order of about 0.01 to about 5.00 mole.

The second method for preparing the novel compounds of the general structural formula V are described in detail below.

Phenylalaninols of structural formula II are reacted with Di-t-butyl dicarbonate to synthesize N-t-butyloxycarbonyl-phenylalaninol represented by the general formula VII: 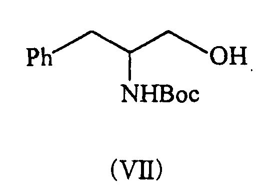 wherein Boc is butyloxycarbonyl group.

Then, the compound of structural formula VII is subjected to treatment with 1,1'-carbonyldiimidazole in an ethereal solution, a halogenated hydrocarbon solution or mixtures thereof, followed by treatment in the presence of an amine base, represented by the following general structural formula VI:

R¹R²NH (VI)

wherein R¹ and R² are the same as defined above, to yield O-carbamoyl -N-t-butyloxycarbonyl-phenylalaninol represented by the general formula VIII: 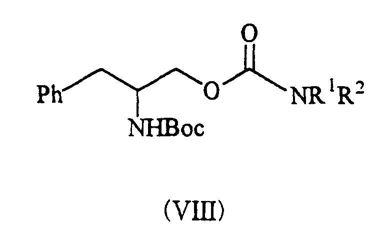 wherein Ph, R¹ and R² are as defined above Then, this intermediate is deprotected by aqueous hydrochloric acid solution. As a result of the deprotection, there is obtained O-carbamoyl-phenylalaninol represented by the general formula V. Without further purification, the compound of formula V may be converted into pharmaceutically acceptable salts I as described above.

This proceduce for preparing the compound of structural formula I is summarized in Reaction Scheme II set forth below.

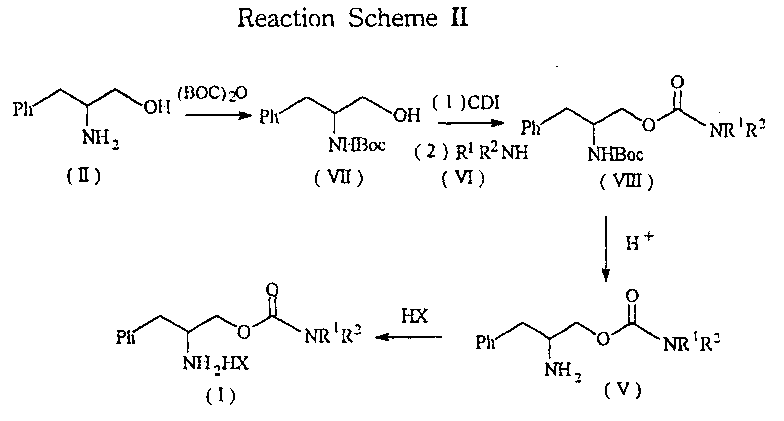

Details of the reaction conditions described in Reaction Scheme II are as follows. In the first step, the concentration of the starting material VII is about 0.005 to about 3 moles with 1,1'-carbonyldiimidazole ranging from about 1.0 to about 2.0 equivalents. This reaction is preferably carried out at a temperature of about -10 to about 70°C. Without purification, the resulting intermediate is treated with from about 1 to about 1,000 equivalents of ammonia at a temperature of about -30 to about 30°C, to give the compound of the general formula VIII. For this carbamoylation, an ethereal solvent such as dichloromethane and chloroform, or mixtures thereof, may be used. The compound of general formula VIII (about 0.005 to about 3 moles) is treated with aqueous 1 to 12 N hydrochloric acid at a temperature of about -10 to about 30°C, followed by neutralization.

In Reaction Scheme II, HX represents an acid capable of forming a pharmacologically useful salt with the basic nitrogen atom.

Representative examples of the compound (V and IX) from Reaction Scheme I and II are shown in Table I.

Table I. Examples of the compound (V and IX) from Reaction Scheme I and II.

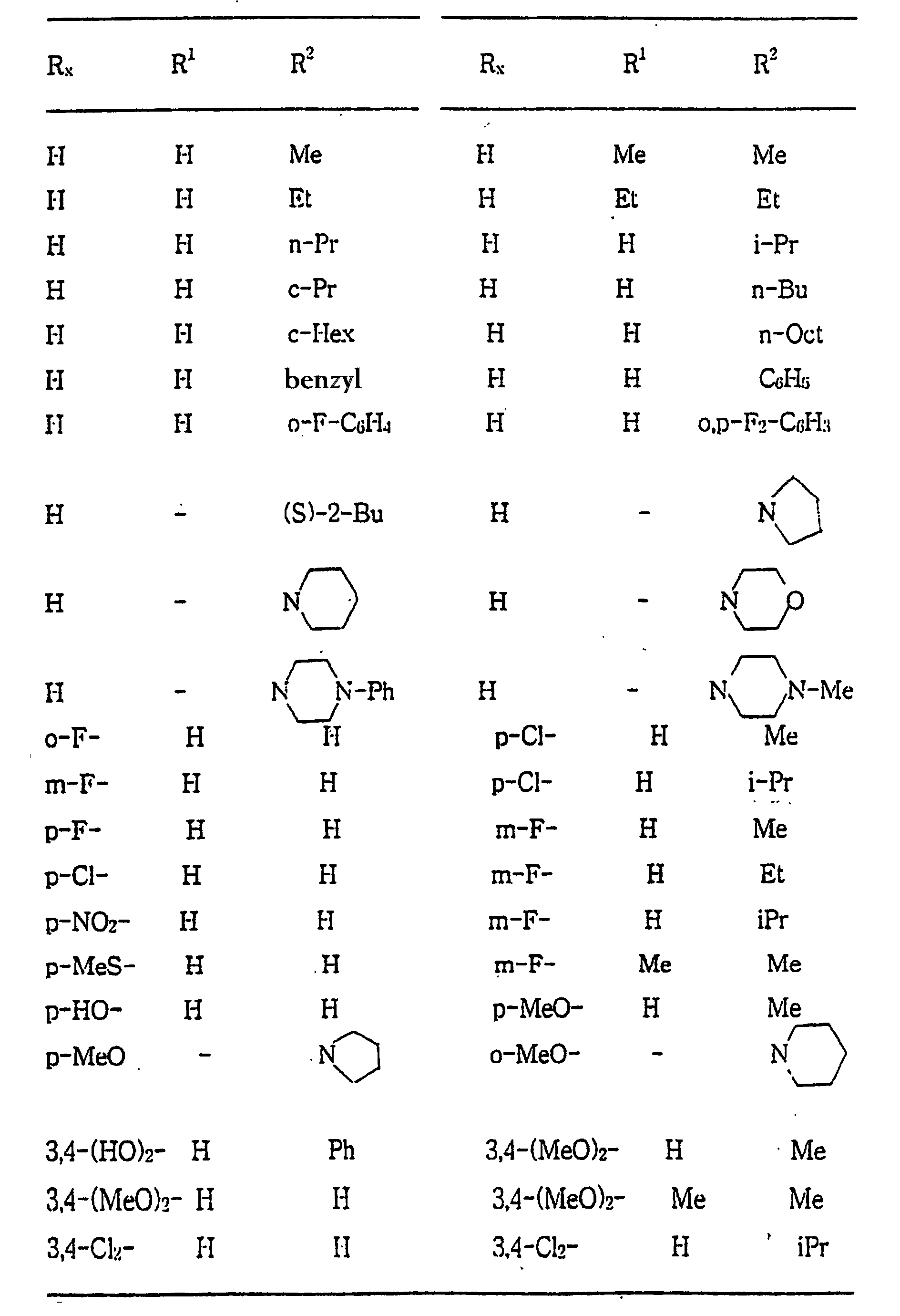

For therapeutic use in medicines for treating pain, depression anxiety, epilepsy, stroke, demential and Parkinson's disease, the compounds of the present invention, alone or in combination with a pharmaceutically acceptable carrier, are administered to patients at a dosage of from 0.7 to 7,000 mg per day. For a normal human adult with a body weight of approximately 70kg, the administration amount is translated into a daily dose of 0.01 to 100 mg per kg of body weight. The specific dosage employed, however, may vary depending upon the requirements of the patient, the severity of the patient's condition and the activity of the compound. The determination of optimum dosages for a particular situation must be determined clinically and is within the skill of the art.

In utilizing the compounds of the present invention for the central nervous system, particularly to treat depression, it is preferred to administer the compounds orally. Since the compounds are well absorbed, when administered orally, it usually will not be necessary to resort to parenteral administration. For oral administration, the compound (I) is preferably combined with a pharmaceutical carrier. The ratio of the carrier to the compound of Structural Formula I is not critical to express the effects of the medicine on the central nervous system, and they can vary considerably depending on whether the composition is to be filled into capsules or formed into tablets. In tableting, various edible pharmaceutical carriers or the mixture thereof can be used. A suitable carrier, for example, is a mixture of lactose, diabasic calcium phosphate and/or corn starch. Other pharmaceutically acceptable ingredients can also be added, including lubricants such as magnesium stearate.

Besides the compound of structural formula I, compositions comprising it are within the scope of the present invention. Furthermore, the present invention includes uses of the compound I and/or the composition.

A better understanding of the present invention may be obtained in light of following examples which are set forth to illustrate, but are not to be construed to limit, the present invention.

### EXAMPLE 1

### Preparation of O-Carbamoyl-N-(t-Butyloxycarbonyl)-o-Fluorophenylalaninol

In a 250 mL flask equipped with magnetic stirrer, N-(t-butyloxycarbonyl)-o- fluorophenylalaninol (0.096 mole, 2.15g) was dissolved in 200ml of THF and was added with 1,1'-carbonyldiimidazole (0.010 mol, 1.62g) at 0°C. The reaction mixture was stirred at room temperature for 2 hours, followed by the injection of ammonia at 0°C for 30 min. Following elevating to room temperature, water was added to terminate the reaction. The organic layer was extracted 3 times with dichloromethane, dried over magnesium sulfate and distilled in vacuo, to give a solid. This was recrystallized in a solution mixture of n-hexane and diethyl ether, to produce 1.93g of O-carbamoyl-N-(t-butyloxycarbonyl)-o-fluorophenylalaninol: Yield 75%.
¹H-NMR(CDCl₃, 200 MHz), ppm(δ): 1.45(s, 9H), 2.88(d, 2H), 4.09(s, 2H), 4.60-4.83(br, 2H), 6.99-7.32(m, 4H)

### EXAMPLE 2

### Preparation of O-Carbamoyl-N-(t-Butyloxycarbonyl)-p-Fluorophenylalaninol

The procedure given in Example 1 was followed using N-(t-butyloxycarbonyl)-p-fluorophenylalaninol as a starting material, instead of N- (t-butyloxycarbonyl)-o-fluorophenylalaninol, to give 2.91g of the title compound. A yield of 88% was obtained.
¹H-NMR(CDCl₃, 200 MHz), ppm(δ): 1.45(s, 9H), 2.68-2.95(m, 2H), 4.02(s, 2H), 4.60-4.90(br, 2H), 6.85-7.29(m, 4H)

### EXAMPLE 3

### Preparation of O-Carbamoyl-N-(t-Butyloxycarbonyl)-p-Nitrophenylalaninol

The procedure given in Example 1 was followed using N-(t-butyloxycarbonyl)-p-nitrophenylalaninol as a starting material, instead of N- (t-butyloxycarbonyl)-o-fluorophenylalaninol, to give 2.66g of the title compound. A yield of 76% was obtained.
¹H-NMR(CDCl₃, 200 MHz), ppm(δ): 1.25(s, 9H), 2.60-2.82(m, 1H), 2.85-3.05(m, 1H), 3.80-4.10(m, 3H), 6.52(s, 1H), 6.90(d, 1H), 7.45(d, 2H), 8.20(d, 2H)

### EXAMPLE 4

### Preparation of O-Carbamoyl-N-(t-Butyloxycarbonyl)-p-(t-Butyloxycarbonyl oxy)-phenylalaninol

The procedure given in Example 1 was followed using N-(t-butyloxycarbonyl)-p-(t-butyloxycarbonyloxy)-phenylalaninol as a starting material, instead of N-(t-butyloxycarbonyl)-o-fluorophenylalaninol, to give 2.55g of the title compound. A yield of 68% was obtained.
¹H-NMR(CDCl₃, 200 MHz), ppm(δ): 1.38(s, 9H), 1.55(s, 9H), 2.70-2.92(m, 2H), 3.68-3.81(m, 1H), 3.98-4.12(m, 3H), 4.68-4.91(br, 2H), 7.01-7.31(m, 4H)

### EXAMPLE 5

### Preparation of O-Carbamoyl-N-Benzyloxycarbonyl-m-Fluorophenylalaninol

In a 100 mL flask equipped with magnetic stirrer, N-benzyloxycarbonyl-m-fluorophenylalaninol (0.007mol, 2.12g) was dissolved in 50 ml of THF and was added with 1,1'-carbonyldiimidazole (0.007 mol, 1.14g) at 0°C. The reaction mixture was stirred at room temperature for 2 hours, followed by the injection of ammonia at 0°C for 30 min. Following elevating to room temperature, water was added to terminate the reaction. The organic layer was extracted 3 times with dichloromethane, dried over magnesium sulfate and distilled in vacuo, to give a solid. This was recrystallized in a solution mixture of n-hexane and diethyl ether, to produce 2.18g of O-carbamoyl-N-benzyloxycarbonyl-m-fluoro phenylalaninol: Yield 91%.
¹H-NMR(CDCl₃, 200 MHz), ppm(δ): 2.49-2.98(m, 2H), 3.69-4.15(m, 4H), 4.80-5.12(m, 2H), 6.35-6.75(br, 2H), 6.80-7.60(m, 9H)

### EXAMPLE 6

### Preparation of O-Carbamoyl-o-Fluorophenylalaninol Hydrochloride

In a 100mL flask equipped with magnetic stirrer, O-carbamoyl -N-(t-butyl oxycarbonyl)-o-fluorophenylalaninol obtained in Example 1 was dissolved in 40 ml of THF and was added with 20ml of 6N aqueous hydrochloric acid solution. The reaction mixture was stirred at room temperature for 8 hours, followed by the neutralization with saturated aqueous potassium carbonate solution. Thereafter, the organic layer was extracted 3 times with dichloromethane, dried over magnesium sulfate and distilled in vacuo, to give a yellowish liquid. This was dissolved in 30 ml of THF and added with anhydrous hydrochloric acid at 0°C, to obtain desirable white precipitates.

To this was added 30ml of anhydrous ether, with the aim of maximizing the precipitation. As a result, 1.22g of the title compound was obtained: Yield 73%.
Melting point: 160-161°C.
¹H-NMR(DMSO-d₆, 200 MHz), ppm(δ): 2.82-3.18(m, 2H), 3.40-3.70(br, 1H), 3.72-4.18(m, 2H), 6.62(s, 2H), 7.08-7.58(m, 4H), 8.45(br, 3H)

### EXAMPLE 7

### Preparation of O-Carbamoyl-p-Fluorophenylalaninol Hydrochloride

The procedure given in Example 6 was followed using O-carbamoyl-N-(t-butyloxycarbonyl)-p-fluorophenylalaninol as a starting material, to give the title compound.
Melting point: 111-113°C
¹H-NMR(DMSO-d₆, 200 MHz), ppm( δ): 2.85-3.20(m, 2H), 3.20-3.60(br, 1H), 3.80-4.20(m, 2H), 6.65(s, 2H), 6.98-7.45(m, 4H), 8.45(br, 3H)

### EXAMPLE 8

### Preparation of O-Carbamoyl-p-Nitrophenylalaninol Hydrochloride

The procedure given in Example 6 was followed using O-carbamoyl-N-(t-butyloxycarbonyl)-p-nitrophenylalaninol obtained in Example 3 as a starting material, to yield the title compound.
¹H-NMR(DMSO-d₆, 200 MHz), ppm(δ): 3.04(d-d, 1H), 3.22(d-d, 2H), 3.67(br, 1H), 3.94(d-d, 1H), 4.06(dd, 1H), 6.63(s, 2H), 7.62(d,2H), 8.24(d, 2H), 8.53(br, 3H)

### EXAMPLE 9

### Preparation of O-Carbamoyl-p-Hydroxy-Phenylalaninol Hydrochloride

The procedure given in Example 6 was followed using O-carbamoyl-N-(t-butyloxycarbonyl)-p-(t-butyloxycarbonyloxy)phenylal aninol obtained in Example 4 as a starting material, to yield the title compound.
Melting point: 213-214°C
¹H-NMR(DMSO-d₆, 200 MHz), ppm(δ): 2.58-3.11(m, 2H), 3.50-3.72(br, 1H), 3.78-4.15(m, 2H), 6.65(s, 2H), 7.10(d, 2H), 8.35(br, 3H), 9.48(s, 1H)

### EXAMPLE 10

### Preparation of O-Carbamoyl-m-Fluorophenylalaninol Hydrochloride

In a 500 mL Parr reactor, O-carbamoyl-N-benzyloxycarbonyl-m-fluoro phenylalaninol (0.006mole, 2.18g) obtained in Example 5 was dissolved in 50mL of anhydrous methanol and added with palladium (carbon powder 10%, 0.10g). Then, the reactor was closed and purged with hydrogen. The reaction was completed in 7 hours under hydrogen pressure of 50 psi at room temperature, which was confirmed on thin layer chromatography. The catalyst was filtered off. Thereafter, the organic layer thus obtained was concentrated through distillation into 1.08g (99%) of pale yellow liquid. The liquid was concentrated through distillation into 1.08g(99%) of pale yellow luquid. The liquid was poured in 30mL of anhydrous THF and cooled to 0°C. Anhydrous hydrochloric acid was then added, to give a desirable white precipitate. Addition of 30 mL of anhydrous ether maximized the precipitation. Filtration provided 1.24g of the title compound.
Melting point: 144-145°C
¹H-NMR(DMSO-d₆, 200 MHz), ppm( δ): 2.85-3.15(m, 2H), 3.50-3.72(br, 1H), 3.82-4.15(m, 2H), 6.65(s, 2H), 7.08-7.28(m, 3H), 7.30-7.51(m, 1H), 8.38(br, 3H).

### EXAMPLE 11

### O-Carbamoyl-3-Chlorophenylalaninol Hydrochloride

The procedure given in Example 6 was followed using O-carbamoyl-N-butyloxycarbonyl-[(3-chloro)phenyl]alaninol as the starting material, to yield the title compound. It possess following properties.
Melting Point: 176-176.3°C
¹H-NMR (DMSO-d₆, 200 MHz), ppm(δ): 2.72-3.18(m, 2H). 3.48-3.62(br, 1H), 3.82-4.15(m, 2H), 6.55(s, 23H), 7.08-7.65(m, 4H), 8.49(br, 3H)

### EXAMPLE 12

### O-Carbamoyl-D-3,4-Dichlorophenylalaninol Hydrochloride

The procedure given in Example 6 was followed using O-carbamoyl-N-butyloxycarbonyl-(D)-[(3,4-dichloro)phenyl]alanin ol as the starting material, to yield the title compound. It possess following properties.
¹H-NMR (DMSO-d₆, 300 MHz), ppm(δ): 2.87-3.08(m, 2H), 3.57(m, 1H), 3.92-4.08(m, 2H), 6.43(br, 2H), 7.28-7.37(m, 1H), 7.50-7.59(m, 2H), 8.37(br, 3H).

### EXAMPLE 13

### O-Carbamoyl-3,4-Dihydroxyphenylalaninol Hydrochloride

The procedure given in Example 6 was followed using O-carbamoyl-N-butyloxycarbonyl-[(3,4-dibutyloxycarbonyloxy)ph enyl]alaninol as the starting material, to yield the title compound. It possess following properties.
Melting Point: 205-206°C
¹H-NMR (DMSO-d₆, 200 MHz); ppm(δ): 2.55-2.95(m, 2H), 3.32-3.69(br, 1H), 3.79-4.18(m, 2H), 6.38-6.96(m, 5H), 8.25(br. 3H), 8.99(br, 2H)

### EXAMPLE 14

### O-(N-Isopropyl)-Carbamoyl-D-4-Chlorophenylalaninol Hydrochloride

The procedure given in Example 6 was followed using O-(N-isopropyl)-carbamoyl-N-butyloxycarbonyl-(D)-[(4-chloro)p henyl]alaninol as the starting material, to yield the title compound. It posses following properties.
Melting Point: 173-175°C
¹H-NMR (DMSO-d₆, 300 MHz), ppm(δ): 2.87-3.12(m, 2H), 3.51-3.65(m, 2H), 3.96-4.11(m, 2H), 6.56(br, 1H), 7.29-7.37(m, 4H), 8.33(br, 3H).

As described hereinbefore, the compounds represented by Structural Formula I were observed to be useful for the prophylaxis and treatment of CNS disorders including pain, depression, anxiety, epilepsy, stroke, demential and Parkinson's disease.

## Claims

1. A racemic or enantiomerically enriehed O-carbamoyl-phenylalaninol compound represented by the following structural formula V: 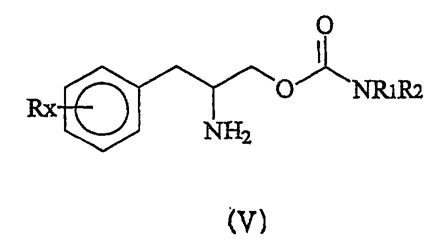 wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, and thioalkoxy containing 1 to 3 carbon atoms, and x is an integer from 1 to 3, with the proviso that R is the same or different when x is 2 or 3, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and R¹ and R² may be joined to form a 5 to 7-membered cyclic moiety which may contain one additional nitrogen atom substituted with a member selected from the group consisting of hydrogen, alkyl, and aryl groups, or one oxygen atom and the pharmaceutically acceptable salts thereof.

2. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is an integer from 1 to 3, with the proviso that R is the same or different when x is 2 or 3, and pharmaceutically acceptable salts thereof.

3. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms and hydroxy, x is an integer from 1 to 3, with the proviso that R is the same or different when x is 2 or 3, and pharmaceutically acceptable salts thereof.

4. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is 1, and pharmaceutically acceptable salts thereof.

5. The O-carbamoyl-phenylalaninol compound represented by the structural formula V, in accordance with claim 1, wherein R¹ and R² are hydrogen, R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is an integer from 1 to 3, with the proviso that R is the same or different when x is 2 or 3, and pharmaceutically acceptable salts thereof.

6. The O-carbamoyl-phenylalaninol compound represented by the structural formula V, in accordance with claim 1, wherein R¹ and R² are hydrogen, R is a member selected from the group consisting of lower alkyl of 1 to 8 carbon atoms, halogen selected from F, Cl, Br and I, alkoxy containing 1 to 3 carbon atoms and hydroxy, x is an integer from 1 to 3, with the proviso that R is the same or different when x is 2 or 3, and pharmaceutically acceptable salts thereof.

7. The O-carbamoyl-phenylalaninol compound represented by the structural formula V, in accordance with claim 1, wherein R¹ and R² are hydrogen, R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is 1, and pharmaceutically acceptable salts thereof.

8. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is an integer from 1 to 3, with the proviso that R is the same or different when x is 2 or 3, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and the pharmaceutically acceptable salts thereof.

9. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms and hydroxy, x is an integer from 1 to 3, with the proviso that R is the same or different when x is 2 or 3, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and the pharmaceutically acceptable salts thereof.

10. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is 1, R¹ and R² may be the same or different from each other and are independently selected from the group consisting of hydrogen, lower alkyl of 1 to 8 carbon atoms, aryl, arylalkyl, cyclic propyl and 5 to 7-membered aliphatic cyclic compounds, and the pharmaceutically acceptable salts thereof.

11. The O-carbamoyl-phenylalaninol compound represented by the structural formula V, in accordance with claim 1, wherein R is a member selected from the group consisting of lower alkyl of 1 to 8. carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is an integer from 1 to 3, with the proviso that R is the same or different when x is 2 or 3, R¹ and R² may be joined to form a 5 to 7-membered cyclic moiety which may contain one additional nitrogen atom substituted with a member selected from the group consisting of hydrogen, alkyl, and aryl groups, or one oxygen atom and the pharmaceutically acceptable salts thereof.

12. The O-carbamoyl-phenylalaninol compound represented by the Structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms and hydroxy, x is an integer from 1 to 3, with the proviso that R is the same or different when x is 2 or 3, R¹ and R² may be joined to form a 5 to 7-membered cyclic moiety which may contain one additional nitrogen atom substituted with a member selected from the group consisting of hydrogen, alkyl, and aryl groups. or one oxygen atom and the pharmaceutically acceptable salts thereof.

13. The O-carbamoyl-phenylalaninol compound represented by the structural formula V, in accordance with claim 1, wherein R is a member selected from the group consisting of lower alkyl of 1 to 8 carbon atoms, halogen selected from F, Cl, Br and I, alkoxy containing 1 to 3 carbon atoms, nitro, hydroxy, trifluoromethyl, or thioalkoxy containing 1 to 3 carbon atoms, x is 1, R¹ and R² may be joined to form a 5 to 7-membered cyclic moiety which may contain one additional nitrogen atom substituted with a member selected from the group consisting of hydrogen, alkyl, and aryl groups, or one oxygen atom and the pharmaceutically acceptable salts thereof.

14. The O-carbamoyl-phenylalaninol compound represented by the structural formula V, in accordance with claim 1, which comprises compounds having the following structural formulas: 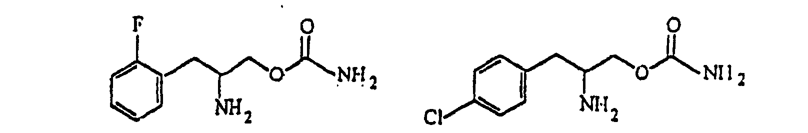 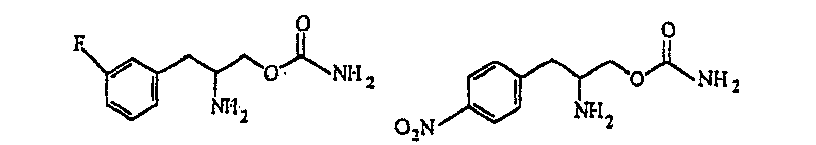 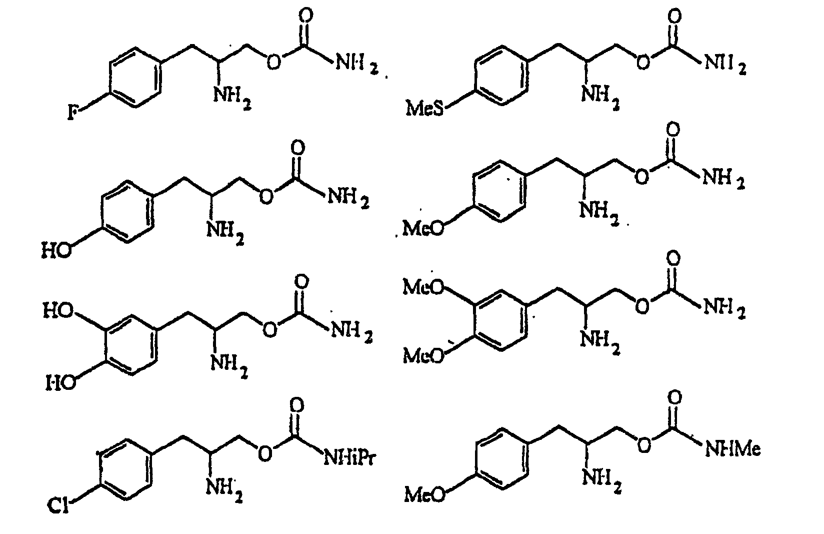 and pharmaceutically acceptable salts thereof.

15. An enantiomerically enriched O-carbamoyl-(D)-phenylalaninol compound represented by the structural formula IX: 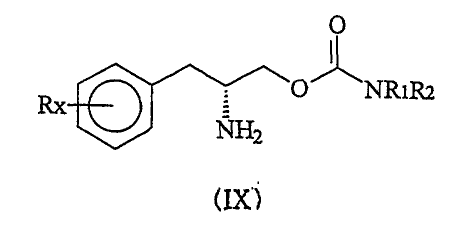 wherein R R¹ and R² are as defined in any one of claims 1 to 13.

16. The O-carbamoyl-(D)-phenylalaninol compound represented by the structural formula IX, in accordance with claim 15, which comprises compounds having the following structural formulas: 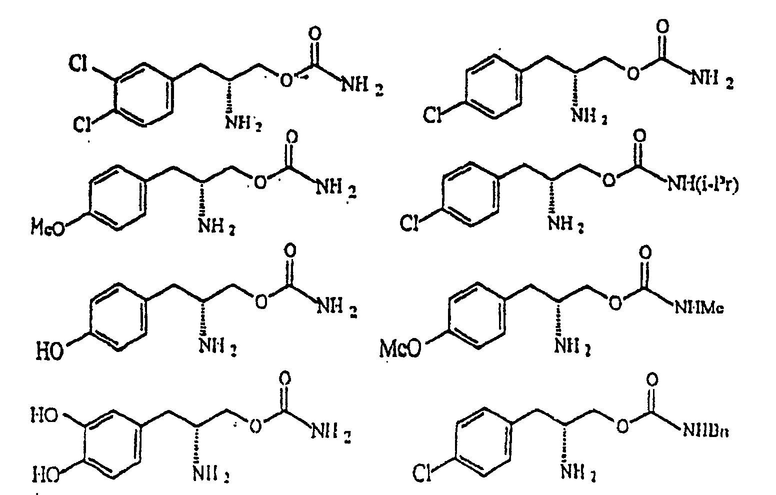 and pharmaceutically acceptable salts thereof.

## Patentansprüche

1. Racemische oder enantiomerenangereicherte O-Carbamoyl-phenylalaninol-Verbindung der folgenden Strukturformel V: 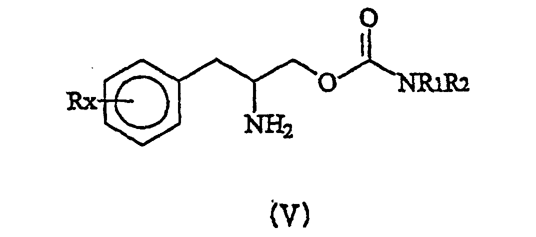 worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl und Thioalkoxy mit 1 - 3 Kohlenstoffatomen ausgewähltes Element ist und x eine ganze Zahl von 1 - 3 ist, wobei R im Falle von x gleich 2 oder 3 gleich oder unterschiedlich ist, R¹ und R² gleich oder voneinander verschieden sein können und unabhängig voneinander aus der Gruppe aus Wasserstoff, Niedrigalkyl mit 1 - 8 Kohlenstoffatomen, Aryl, Arylalkyl, cyclischem Propyl und 5- bis 7gliedrigen aliphatischen cyclischen Verbindungen ausgewählt sind und R¹ und R² unter Bildung einer 5- bis 7gliedrigen cyclischen Baueinheit, die ein zusätzliches Stickstoffatom, das mit einem aus der Gruppe aus Wasserstoff, Alkyl- und Arylgruppen ausgewählten Element substituiert ist, oder ein Sauerstoffatom enthalten kann, miteinander verbunden sein können, sowie die pharmazeutisch akzeptablen Salze derselben.

2. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist, x eine ganze Zahl von 1 - 3 ist, wobei R im Falle von x gleich 2 oder 3 gleich oder unterschiedlich ist,
sowie pharmazeutisch akzeptable Salze derselben.

3. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen und Hydroxy ausgewähltes Element ist, x eine ganze Zahl von 1 - 3 ist, wobei R im Falle von x gleich 2 oder 3 gleich oder unterschiedlich ist, sowie pharmazeutisch akzeptable Salze derselben.

4. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist, x gleich 1 ist,
sowie pharmazeutisch akzeptable Salze derselben.

5. O-Carbamoyl-phenylalaninol-Verbindung der Strukturformel V gemäß Anspruch 1, worin R¹ und R² Wasserstoff sind, R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist, x eine ganze Zahl von 1 - 3 ist, wobei R im Falle von x gleich 2 oder 3 gleich oder unterschiedlich ist, sowie pharmazeutisch akzeptable Salze derselben.

6. O-Carbamoyl-phenylalaninol-Verbindung der Strukturformel V gemäß Anspruch 1, worin R¹ und R² Wasserstoff sind, R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen und Hydroxy ausgewähltes Element ist, x eine ganze Zahl von 1 - 3 ist, wobei R im Falle von x gleich 2 oder 3 gleich oder unterschiedlich ist,
sowie pharmazeutisch akzeptable Salze derselben.

7. O-Carbamoyl-phenylalaninol-Verbindung der Strukturformel V gemäß Anspruch 1, worin R¹ und R² Wasserstoff sind, R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist, x gleich 1 ist,
sowie pharmazeutisch akzeptable Salze derselben.

8. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist und x eine ganze Zahl von 1 - 3 ist, wobei R im Falle von x gleich 2 oder 3 gleich oder unterschiedlich ist,
R¹ und R² gleich oder voneinander verschieden sein können und unabhängig voneinander aus der Gruppe aus Wasserstoff, Niedrigalkyl mit 1 - 8 Kohlenstoffatomen, Aryl, Arylalkyl, cyclischem Propyl und 5- bis 7gliedrigen aliphatischen cyclischen Verbindungen ausgewählt sind,
sowie die pharmazeutisch akzeptablen Salze derselben.

9. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen und Hydroxy ausgewähltes Element ist und x eine ganze Zahl von 1 - 3 ist, wobei R im Falle von x gleich 2 oder 3 gleich oder unterschiedlich ist, R¹ und R² gleich oder voneinander verschieden sein können und unabhängig voneinander aus der Gruppe aus Wasserstoff, Niedrigalkyl mit 1 - 8 Kohlenstoffatomen, Aryl, Arylalkyl, cyclischem Propyl und 5- bis 7gliedrigen aliphatischen cyclischen Verbindungen ausgewählt sind,
sowie die pharmazeutisch akzeptablen Salze derselben.

10. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist und x gleich 1 ist,
R¹ und R² gleich oder voneinander verschieden sein können und unabhängig voneinander aus der Gruppe aus Wasserstoff, Niedrigalkyl mit 1 - 8 Kohlenstoffatomen, Aryl, Arylalkyl, cyclischem Propyl und 5- bis 7gliedrigen aliphatischen cyclischen Verbindungen ausgewählt sind, sowie die pharmazeutisch akzeptablen Salze derselben.

11. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist und x eine ganze Zahl von 1 - 3 ist, wobei R im Falle von x gleich 2 oder 3 gleich oder unterschiedlich ist,
R¹ und R² unter Bildung einer 5- bis 7gliedrigen cyclischen Baueinheit, die ein zusätzliches Stickstoffatom, das mit einem aus der Gruppe aus Wasserstoff, Alkyl- und Arylgruppen ausgewählten Element substituiert ist, oder ein Sauerstoffatom enthalten kann, miteinander verbunden sein können,
sowie die pharmazeutisch akzeptablen Salze derselben.

12. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen und Hydroxy ausgewähltes Element ist und x eine ganze Zahl von 1 - 3 ist, wobei R im Falle von x gleich 2 oder 3 gleich oder unterschiedlich ist, R¹ und R² unter Bildung einer 5- bis 7gliedrigen cyclischen Baueinheit, die ein zusätzliches Stickstoffatom, das mit einem aus der Gruppe aus Wasserstoff, Alkyl- und Arylgruppen ausgewählten Element substituiert ist, oder ein Sauerstoffatom enthalten kann, miteinander verbunden sein können,
sowie die pharmazeutisch akzeptablen Salze derselben.

13. O-Carbamoyl-phenylalaninol-Verbindung der Strukturformel V gemäß Anspruch 1, worin R ein aus der Gruppe aus Niedrigalkyl mit 1 - 8 Kohlenstoffatomen, Halogen, das aus F, Cl, Br und I ausgewählt ist, Alkoxy mit 1 - 3 Kohlenstoffatomen, Nitro, Hydroxy, Trifluormethyl oder Thioalkoxy mit 1 - 3 Kohlenstoffatomen ausgewähltes Element ist und x gleich 1 ist,
R¹ und R² unter Bildung einer 5- bis 7gliedrigen cyclischen Baueinheit, die ein zusätzliches Stickstoffatom, das mit einem aus der Gruppe aus Wasserstoff, Alkyl- und Arylgruppen ausgewählten Element substituiert ist, oder ein Sauerstoffatom enthalten kann, miteinander verbunden sein können,
sowie die pharmazeutisch akzeptablen Salze derselben.

14. O-Carbamoyl-phenylalaninol-Verbindung der Strukturformel V gemäß Anspruch 1, die Verbindungen der folgenden Strukturformeln; 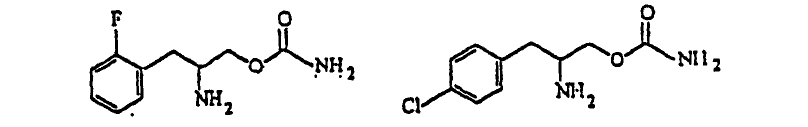 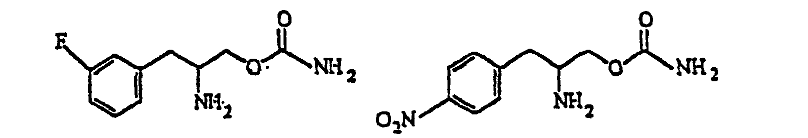 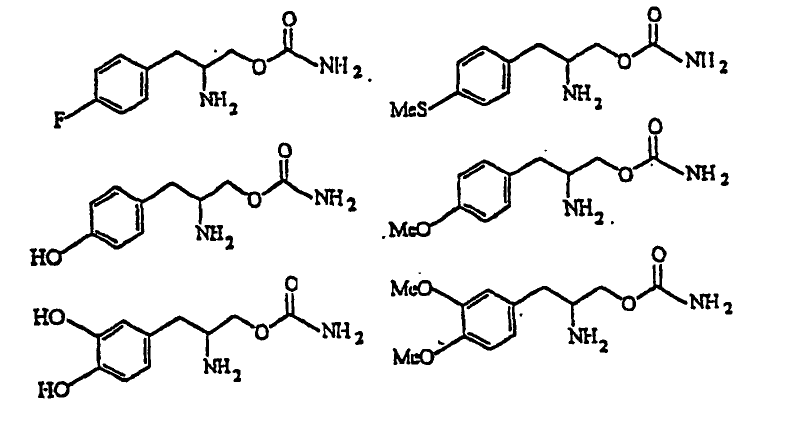 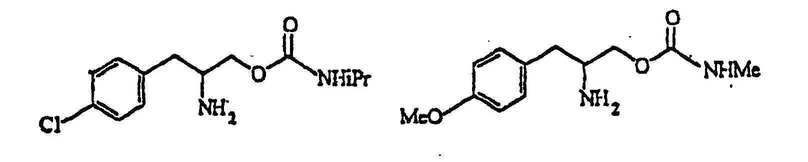 umfasst, sowie pharmazeutisch akzeptable Salze derselben.

15. Enantiomerenangereicherte O-Carbamoyl-(D)-phenylalaninol-Verbindung der Strukturformel IX 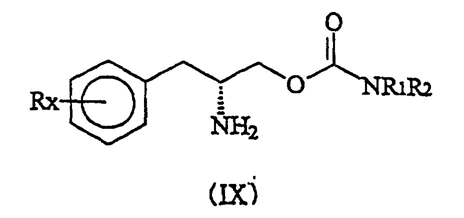 worin R, R¹ und R² wie in einem der Ansprüche 1 - 13 definiert sind.

16. O-Carbamoyl-(D)-phenylalaninol-Verbindung der Strukturformel IX gemäß Anspruch 15, die Verbindungen der folgenden Strukturformeln: 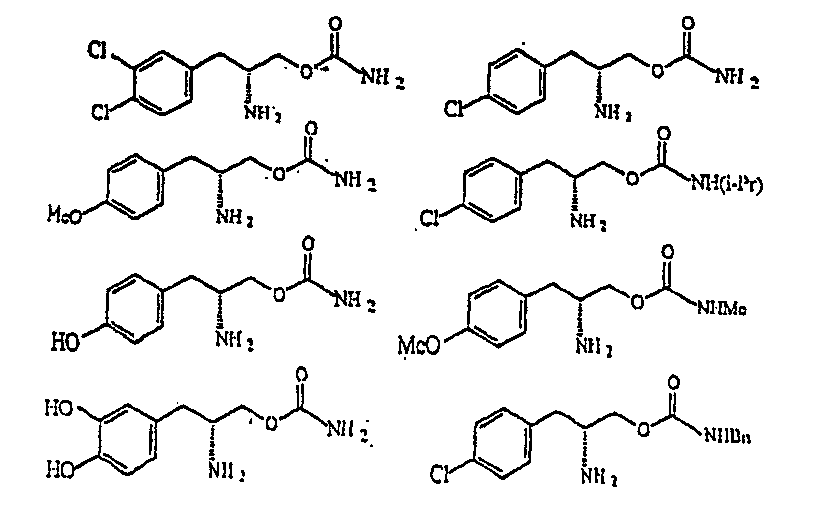 umfasst, sowie pharmazeutisch akzeptable Salze derselben.

## Revendications

1. Composé d'O-carbamoyl-phénylalaninol racémique ou énantiomériquement enrichi représenté par la formule de structure V suivante : 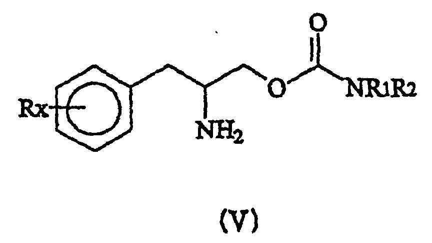 dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, et thioalcoxy contenant 1 à 3 atomes de carbone, et x est un nombre entier de 1 à 3, à condition que R soit identique ou différent quand x vaut 2 ou 3, R¹ et R² peuvent être identiques ou différents l'un de l'autre et sont, indépendamment l'un de l'autre, choisis dans le groupe formé par un atome d'hydrogène, un groupe alkyle de 1 à 8 atomes de carbone, aryle, arylalkyle, cyclopropyle, et des composés cycloaliphatiques de 5 à 7 éléments, et R¹ et R² peuvent être réunis pour former une fraction cyclique de 5 à 7 éléments qui peut contenir un atome d'azote supplémentaire substitué avec un élément choisi dans le groupe formé par un atome d'hydrogène, des groupes alkyle et aryle, ou un atome d'oxygène, et ses sels acceptables sur le plan pharmaceutique.

2. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br, I, un groupe alcoxy contenant 1 à 3 atomes de carbone, nitro, hydroxy, trifluorométhyle, ou thioalcoxy contenant 1 à 3 atomes de carbone, x est un nombre entier de 1 à 3, à condition que R soit identique ou différent quand x vaut 2 ou 3, et ses sels acceptables sur le plan pharmaceutique.

3. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone et hydroxy, x est un nombre entier de 1 à 3, à condition que R soit identique ou différent quand x vaut 2 ou 3, et ses sels acceptables sur le plan pharmaceutique.

4. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x vaut 1, et ses sels acceptables sur le plan pharmaceutique.

5. Composé d'O-carbamoyl-phénylalaninol représenté par la formule de structure V, selon la revendication 1, dans laquelle R¹ et R² sont des atomes d'hydrogène, R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant à 1 à 3 atomes de carbone, x est un nombre entier de 1 à 3, à condition que R soit identique ou différent quand x vaut 2 ou 3, et ses sels acceptables sur le plan pharmaceutique.

6. Composé d'O-carbamoyl-phénylalaninol représenté par la formule de structure V, selon la revendication 1, dans laquelle R¹ et R² sont des atomes d'hydrogène, R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone et hydroxy, x est un nombre entier de 1 à 3, à condition que R soit identique ou différent quand x vaut 2 ou 3, et ses sels acceptables sur le plan pharmaceutique.

7. Composé d'O-carbamoyl-phénylalaninol représenté par la formule de structure V, selon la revendication 1, dans laquelle R¹ et R² sont des atomes d'hydrogène, R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x vaut 1, et ses sels acceptables sur le plan pharmaceutique.

8. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x est un nombre entier de 1 à 3, à condition que R soit identique ou différent quand x vaut 2 ou 3, R¹ et R² peuvent être identiques ou différents l'un de l'autre et sont, indépendamment l'un de l'autre, choisis dans le groupe formé par un atome d'hydrogène, un groupe alkyle inférieur de 1 à 8 atomes de carbone, aryle, arylalkyle, cyclopropyle et des composés cycloaliphatiques de 5 à 7 éléments, et ses sels acceptables sur le plan pharmaceutique.

9. Composé d'O-carbamoyl-phénylalaninol représenté par la formule de structure V, selon la revendication 1, dans laquelle R et un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone et hydroxy, x est un nombre entier de 1 à 3, à condition que R soit identique ou différent quand x vaut 2 ou 3, R¹ et R² peuvent être identiques ou différents l'un de l'autre et sont, indépendamment l'un de l'autre, choisis dans le groupe formé par un atome d'hydrogène, un groupe alkyle inférieur de 1 à 8 atomes de carbone, aryle, arylalkyle, cyclopropyle et des composés cycloaliphatiques de 5 à 7 éléments, et ses sels acceptables sur le plan pharmaceutique.

10. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x vaut 1, R¹ et R² peuvent être identiques ou différents l'un de l'autre et sont, indépendamment l'un de l'autre, choisis dans le groupe formé par un atome d'hydrogène, un groupe alkyle de 1 à 8 atomes de carbone, aryle, arylalkyle, cyclopropyle, et des composés cycloaliphatiques de 5 à 7 éléments, et ses sels acceptables sur le plan pharmaceutique.

11. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x est un nombre entier de 1 à 3, à condition que R soit identique ou différent quand x vaut 2 ou 3, R¹ et R² peuvent être réunis pour former une fraction cyclique de 5 à 7 éléments qui peut contenir un atome d'azote supplémentaire substitué avec un élément choisi dans le groupe formé par un atome d'hydrogène, des groupes alkyle et aryle, ou un atome d'oxygène, et ses sels acceptables sur le plan pharmaceutique.

12. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone et hydroxy, x est un nombre entier de 1 à 3, à condition que R soit identique ou différent quand x vaut 2 ou 3, R¹ et R² peuvent être réunis pour former une fraction cyclique de 5 à 7 éléments qui peut contenir un atome d'azote supplémentaire substitué avec un élément choisi dans le groupe formé par un atome d'hydrogène, des groupes alkyle et aryle, ou un atome d'oxygène, et ses sels acceptables sur le plan pharmaceutique.

13. Composé d'O-carbamoyl-phénylalaninol représenté par la formule de structure V, selon la revendication 1, dans laquelle R est un élément choisi dans le groupe formé par un groupe alkyle inférieur de 1 à 8 atomes de carbone, un atome d'halogène choisi parmi F, Cl, Br et I, un groupe alcoxy contenant 1 à 3 atomes de carbone, nitro, hydroxy, trifluorométhyle, ou thioalcoxy contenant 1 à 3 atomes de carbone, x vaut 1, R¹ et R² peuvent être réunis pour former une fraction cyclique de 5 à 7 éléments qui peut contenir un atome d'azote supplémentaire substitué avec un élément choisi dans le groupe formé par un atome d'hydrogène, des groupes alkyle et aryle, ou un atome d'oxygène, et ses sels acceptables sur le plan pharmaceutique.

14. Composé d'O-carbamoyl-phénylalaninol représenté par la formule de structure V, selon la revendication 1, qui comprend des composés possédant les formules de structure suivantes : 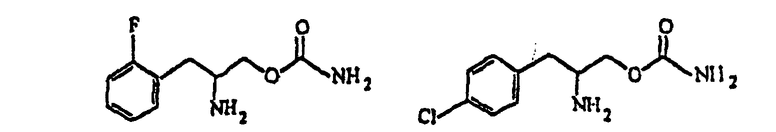 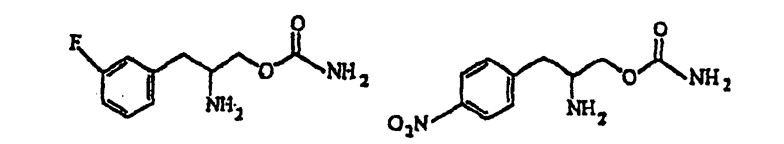 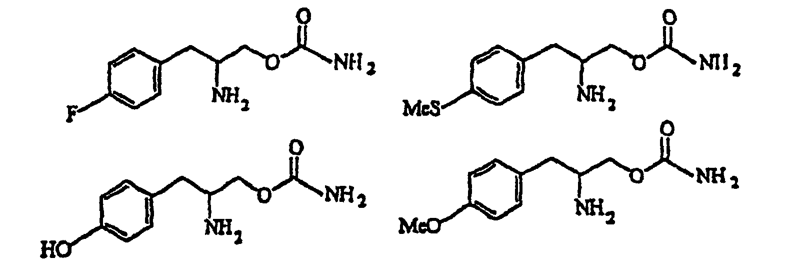 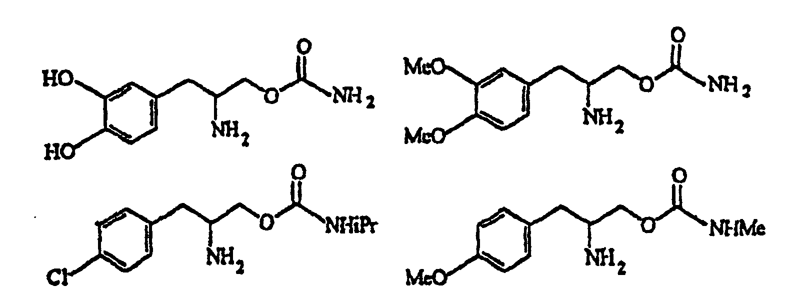 et ses sels acceptables sur le plan pharmaceutique.

15. Composé d'O-carbamoyl-(D)-phénylalaninol énantiomériquement enrichi représenté par la formule de structure IX : 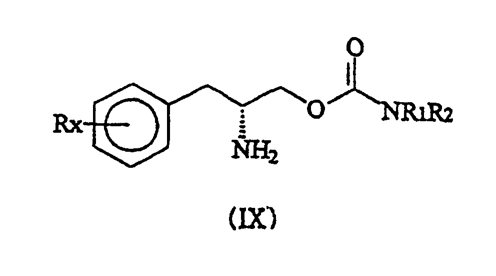 dans laquelle R, R¹ et R² sont tels que définis dans l'une quelconque des revendications 1 à 13.

16. Composé d'O-carbamoyl-(D)-phénylalaninol représenté par la formule de structure IX, selon la revendication 15, qui comprend des composés possédant les formules de structure suivantes : 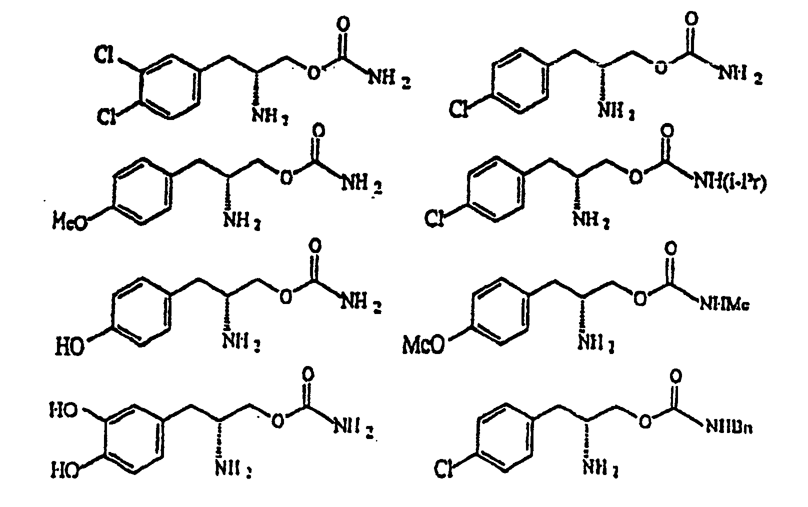 et ses sels acceptables sur le plan pharmaceutique.
